# FASCICULE DE BREVET EUROPEEN

(11) **EP 0 544 581 B1**
(45) Date de publication et mention de la délivrance du brevet: **12.06.1996**
(21) Numéro de dépôt: 92403158.6
(22) Date de dépôt: 24.11.1992
(51) Int. Cl.: A61M 39/00, A61M 39/04

(54) **Connecteur composite unitaire pour circuit de liquide notamment à usage médical**
Einstückiges Flüssigkeitskreislaufverbindungselement, insbesondere für medizinischen Gebrauch
One piece connector for fluid circuits, especially for medical use

(30) Priorité: 25.11.1991 FR 9114492
(43) Date de publication de la demande: 02.06.1993
(73) Titulaire: Société VYGON, F-95440 Ecouen (FR)
(72) Inventeur: Brinon, Thierry, F-95440 Ecouen (FR)
(74) Mandataire: Schrimpf, Robert

(56) Documents cités:
- EP-A- 0 309 771
- DE-C- 3 303 073
- US-A- 3 986 508
- US-A- 4 512 766
- US-A- 4 998 927
- US-A- 5 006 114

## Description

L'invention concerne un connecteur composite unitaire pour circuit de liquide, notamment à usage médical.

L'expression "unitaire" signifie que le connecteur est constitué par un assemblage permanent, au contraire de certains connecteurs antérieurs constitués d'au moins deux ensembles séparés que l'on assemble au moment d'établir la connection.

Elle concerne plus particulièrement un connecteur du type qui comprend des moyens qui constituent une chambre tubulaire entre un raccord amont et un raccord aval situés à des extrémités opposées de la chambre et fixes l'un par rapport à l'autre, ledit raccord aval constituant un passage; une aiguille creuse fixe dans la chambre et apte à faire communiquer le raccord amont avec le raccord aval ; un bouchon apte à être traversé par l'aiguille, monté dans le passage du raccord aval de façon à pouvoir coulisser entre une position aval stable d'obturation où il obture ledit passage et n'est pas traversé par l'aiguille et une position amont où il est traversé par ladite aiguille et vers laquelle il peut être poussé par un organe introduit depuis l'extérieur du connecteur dans ledit passage, et un moyen situé dans la chambre et qui repousse élastiquement le bouchon vers la position stable d'obturation, la matière du bouchon étant telle que le bouchon recouvre ses propriétés d'obturation lorsqu'il n'est plus traversé par l'aiguille.

Un tel connecteur composite unitaire est notamment utile dans le domaine médical, par exemple pour introduire un liquide dans le corps d'un patient et diverses réal¡sations en ont été décrites, notamment dans les documents EP 0309771 et US-A-4 998 927.

Ces publications mettent en évidence qu'il est difficile d'obtenir un connecteur composite unitaire qui soit d'un coût de fabrication acceptable, qui assure un raccordement fiable et efficace et qui soit exempt de risques de contamination.

Un but de l'invention est de fournir un nouveau connecteur composite unitaire du type décrit ci-dessus, apte à réaliser un raccordement fiable et efficace, qui soit d'un coût de fabrication acceptable, notamment si le connecteur doit être jeté après usage et qui ne présente pas de risques de contamination du circuit liquide, notamment dans la région de son raccord aval.

On y parvient selon l'invention par le fait que ledit passage du raccord aval a une ouverture de sortie totalement remplie par la matière du bouchon lorsque ce dernier est en position stable d'obturation, ce qui évite tout volume mort autour du bouchon dans cette ouverture et donc les risques de contamination qui seraient liés à la présence d'un volume mort et en ce que le moyen qui repousse le bouchon vers sa position d'obturation est un ressort.

Le fait d'utiliser un ressort pour repousser le bouchon, alors que les antérirités utilisent une déformation élastique d'une partie tubulaire du bouchon est très important car il permet de choisir à volonté la force de rappel du ressort et d'utiliser un bouchon introduit à force dans le raccord aval en sorte que la compression latérale du bouchon dans le raccord garantisse une bonne étanchéité, le ressort choisi ayant une force suffisante pour pousser le bouchon dans le raccord malgré le frottement du bouchon dans le raccord du à la compression latérale du bouchon.

On décrira ci-après différentes réalisations d'un tel connecteur, à titre d'exemples préférés mais non limitatifs, en référence aux figures du dessin joint sur lequel :
- la figure 1 est une vue éclatée des pièces constitutives d'un connecteur selon l'invention ;
- la figure 2 est une coupe axiale du connecteur en état d'obturation;
- la figure 3 est une coupe axiale du connecteur en état de non-obturation;
- la figure 4 est une coupe axiale d'une variante de connecteur selon l'invention, pour trois positions du bouchon (figs 4A, 4B, 4C) ;
- les figures 5 et 6 sont des vues de face de l'extrémité du bouchon dans le raccord aval dans deux exemples de réalisation ;
- les figures 7 à 12 sont des coupes axiales du bouchon selon différents exemples de réalisation, et
- les figures 13 à 20 sont des coupes montrant différentes réalisations de connecteurs conformes à l'invention.

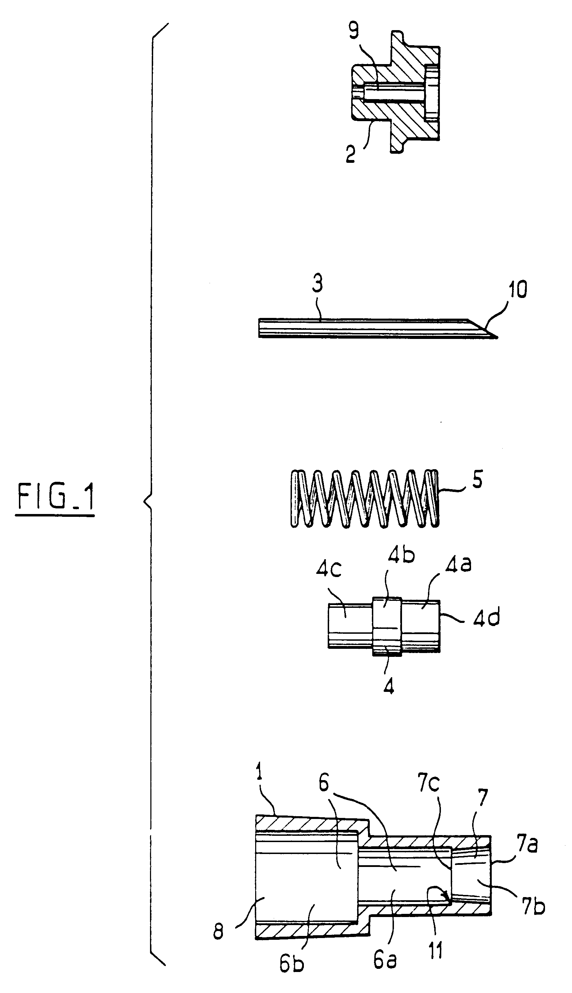

La figure 1 montre les parties constitutives d'un premier exemple de réalisation d'un connecteur de l'invention.

Ce connecteur est un corps rigide constitué par la combinaison de cinq pièces, à savoir :
- un carter 1 non déformable,
- un raccord amont 2,
- une aiguille 3,
- un bouchon d'obturation 4, et
- un ressort hélicoïdal 5.

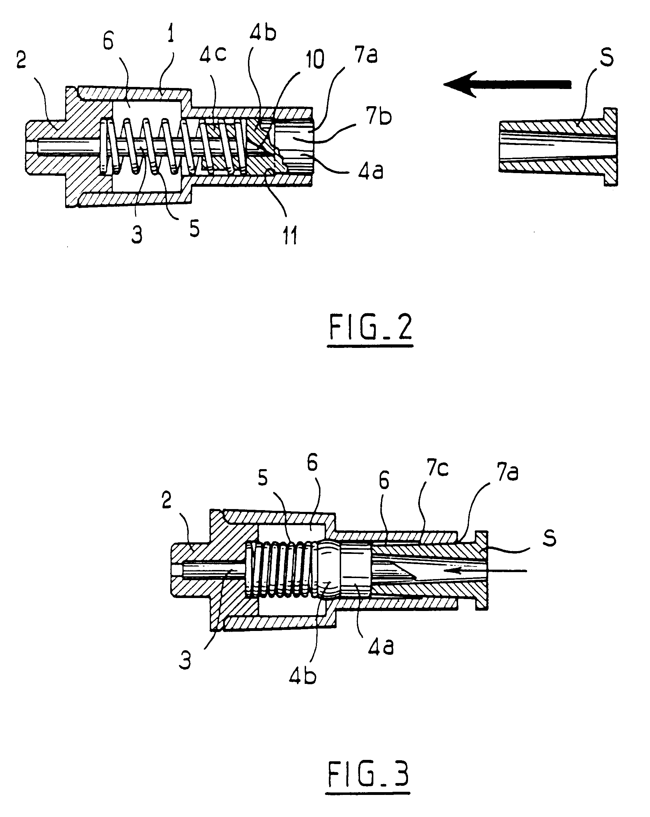

Ces pièces sont assemblées en un ensemble unitaire (figure 2) tenant compte des particularités suivantes.

Le carter 1 délimite une chambre tubulaire 6 entre une extrémité aval 7 qui est conformée pour constituer un raccord aval et une extrémité amont 8 qui est conformée pour recevoir le raccord amont 2. Dans cet exemple, la chambre 6 comporte deux zones 6a, 6b de diamètres différents.

Le raccord amont 2 présente en canal central 9 qui reçoit à friction l'aiguille 3 en sorte que lorsque ce raccord est monté sur le carter, l'aiguille pénètre axialement dans la chambre en direction du raccord aval.

Le raccord aval 7 constitue un passage 7b de forme tronconique, l'ouverture de sortie 7a de ce passage ayant un diamètre légèrement plus grand que celui de son ouverture d'entrée 7c. Ce type de passage est conçu de façon en soi connue pour l'introduction d'un embout 12 a conicité mâle correspondante.

Le bouchon 4, la chambre 6 et le passage 7b sont conformés pour que le bouchon puisse coulisser dans la chambre et le passage avec étanchéité latérale, entre une position aval (fig. 2) et une position amont (fig. 3).

De fait, le bouchon est un bloc cylindrique en matériau élastomère qui présente une extrémité aval 4a apte à coulisser dans le passage 7b avec étanchéité latérale et une extrémité amont 4b de plus grand diamètre apte à coulisser dans la chambre 6 avec étanchéité latérale. L'extrémité amont est précédée d'une queue 4c qui sert au montage du ressort 5, lequel vient en butée contre l'extrémité amont.

Dans la position aval (fig. 2), l'aiguille pénètre dans le bouchon mais l'ouverture biseautée 10 de l'aiguille reste noyée dans le bouchon dont la matière obture cette ouverture. Le bouchon est donc obturateur puisqu'il n'est pas traversé de part en part et il interdit la communication entre les raccords amont et aval.

Dans la position amont (fig. 3), le bouchon a reculé vers l'aiguille jusqu'à dégager totalement le passage 7b du raccord aval et être totalement traversé par l'ouverture biseautée de l'aiguille, qui se trouve alors au-delà du bouchon : dans cette position, les raccords amont et aval communiquent par l'aiguille.

Le ressort 5 est logé dans la chambre 6 entre le raccord amont 2 sur lequel il prend appui et le bouchon 4 qu'il pousse vers sa position aval où le bouchon se trouve en butée contre un épaulement constitué par un rebord annulaire interne 11 de la chambre.

Le ressort est choisi pour autoriser une course du bouchon suffisante pour que le bouchon puisse reculer contre l'action du ressort jusque dans la position amont qui convient.

Ce recul est provoqué par exemple par l'introduction d'un embout S dans le passage conique du raccord aval, par exemple l'extrémité d'une seringue (figures 2 et 3).

Le passage du raccord aval et l'embout présentent des conicités respectivement femelle et mâle qui permettent le maintien par friction de l'embout dans le raccord.

Si l'on retire l'embout, le bouchon revient automatiquement en position d'obturation sous l'action du ressort. La matière du bouchon est telle que le bouchon recouvre ses propriétés d'obturation lorsqu'il n'est plus traversé par l'aiguille. De tels matériaux, sont connus dans cette application et n'ont pas besoin d'être décrits en détails.

Le bouchon en position d'obturation remplit intégralement l'ouverture de sortie 7a du passage conique du raccord aval et, avantageusement, le bouchon présente une extrémité légèrement bombée en saillie hors de cette ouverture de sortie du raccord (figure 2).

On évite ainsi les risques d'introduction d'éléments contaminants dans le passage du raccord lorsque le bouchon est en position d'obturation.

Le raccord amont 2 est de tout type approprié et constitue généralement un raccord mâle.

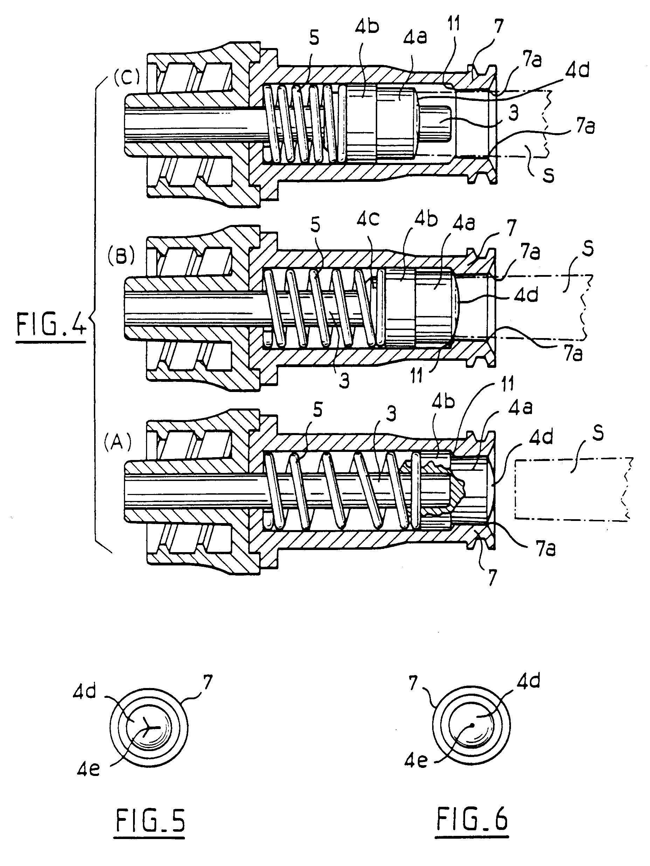

La fig. 4 montre une réalisation dans laquelle l'aiguille 3 est un simple tube dont l'extrémité est noyée dans la matière du bouchon et ne comporte pas de biseau et dans laquelle le bouchon 4 comporte une extrémité aval 4a munie d'une préperforation longitudinale 4e en sorte qu'au repos, c'est-à-dire lorsque cette extrémité n'est pas sollicitée par l'aiguille, la préperforation 4e est fermée sur elle-même (fig. 4A) tandis que sous la sollicitation de l'aiguille lorsque le bouchon est poussé vers l'aiguille par un élément extérieur, par exemple l'extrémité d'une seringue S, la préperforation s'ouvre progressivement pour laisser passer l'aiguille (fig 4B et 4C) de façon en soi connue.

La préperforation a toute forme désirée. Par exemple elle a en section droite transversale la forme d'une croix (fig. 5) ou d'un point (fig. 6).

Le bouchon représenté sur la fig. 4 présente une extrémité cylindrique 4a dont le diamètre est supérieur au diamètre de l'ouverture de sortie 7a du passage 7b, lequel est supérieur à celui de l'ouverture d'entrée 7c de ce passage : il en résulte que le ressort 5 doit être choisi avec une force suffisante pour pousser à force le bouchon vers sa position d'obturation (fig. 4A). L'extrémité du bouchon est ainsi comprimée latéralement dans le raccord aval 7 ce qui assure l'étanchéité latérale du raccord.

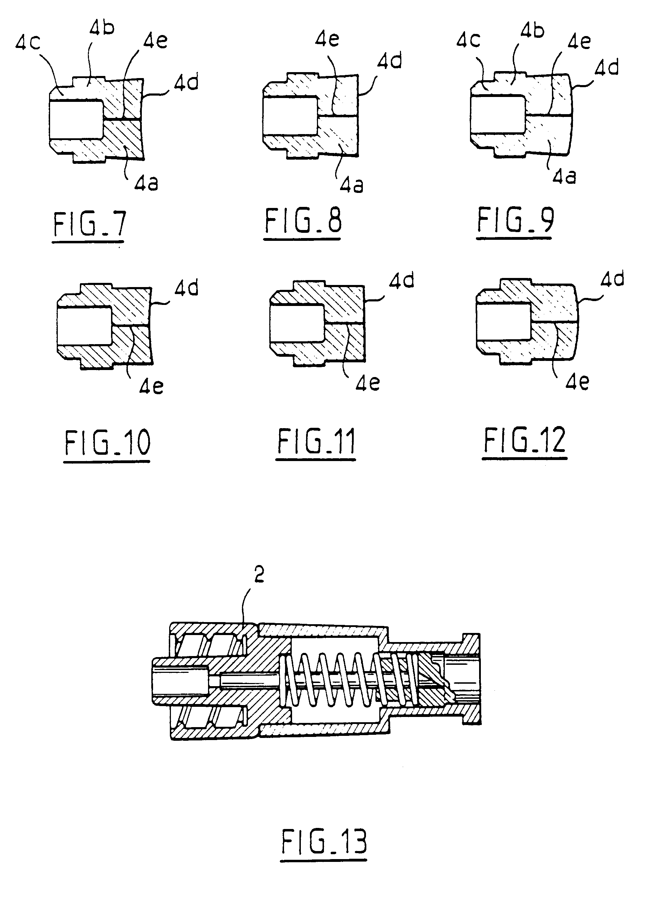

Les fig. 7 à 9 montrent des réalisations dans lesquelles le bouchon présente une face avant 4d, concave (fig. 7), plate (fig. 8) ou convexe (fig 9) et une extrémité 4a de forme tronconique décroissante à partir de la face avant 4d. La conicité de cette extrémité correspond sensiblement à la conicité interne du raccord aval 7.

Les fig. 10 à 12 montrent des réalisations dans lesquelles le bouchon présente une face avant 4d concave (fig. 10), plate (fig. 11) ou convexe (fig. 12) mais où l'extrémité du bouchon 4a est cylyndrique.

Toutes ces réalisations présentent des avantages particuliers soit dans la facilité de fabrication, soit dans les facilités de désinfection, soit dans la qualité de l'étanchéité obtenue. Ainsi une face avant convexe (figs 2, 4, 9, 12) facilite l'introduction à force du bouchon dans le passage du raccord aval sous la poussée du ressort, une face avant concave (figs. 7, 10) présente une meilleure possibilité de décontamination, une forme conique (figs 7 -9) réduit les forces de frottement lorsque le bouchon arrive en fin de course sous la poussée du ressort, c'est-à-dire au moment où la force du ressort est devenue plus faible.

L'invention n'est pas limitée à un choix particulier pour les raccords amont et aval, ce choix dépendant des conditions d'utilisation du connecteur.

Les figures 13 et suivantes montrent, à titre d'exemples non limitatifs, des connecteurs conformes à l'invention comportant divers raccords. Certaines figures sont doubles et montrent respectivement le connecteur au repos (absence de communication) ou en service (communication).

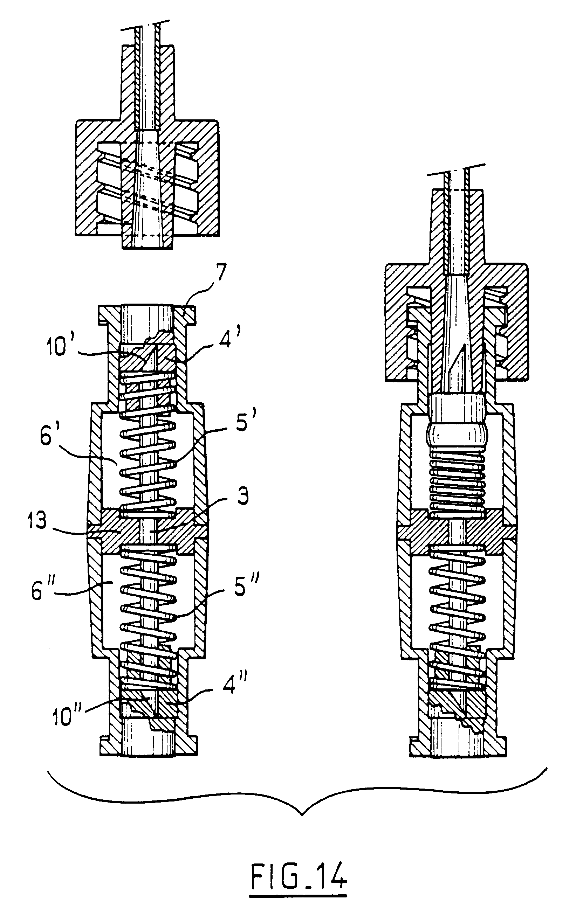

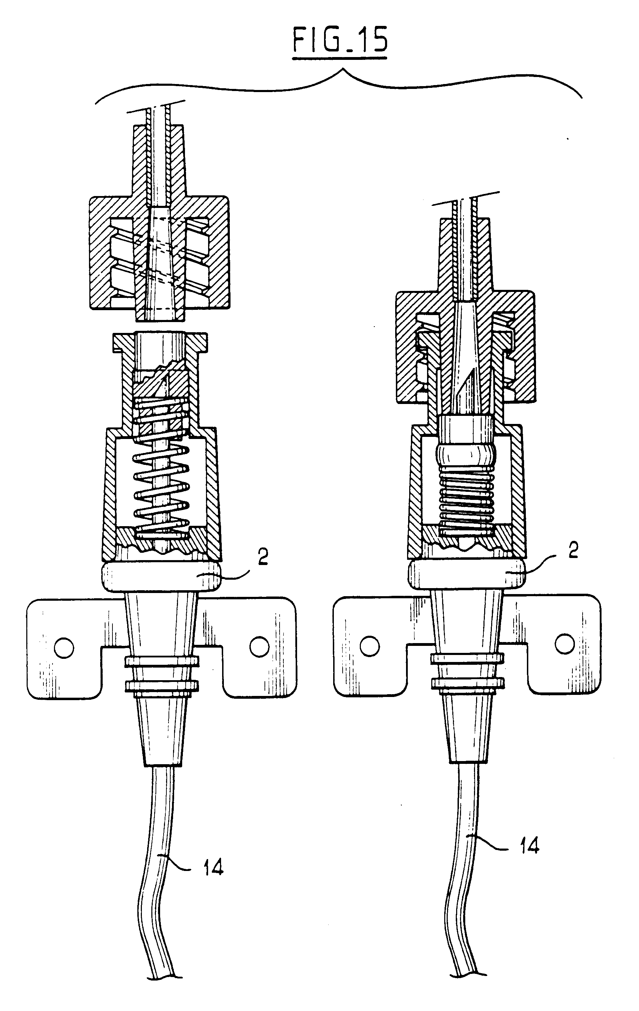

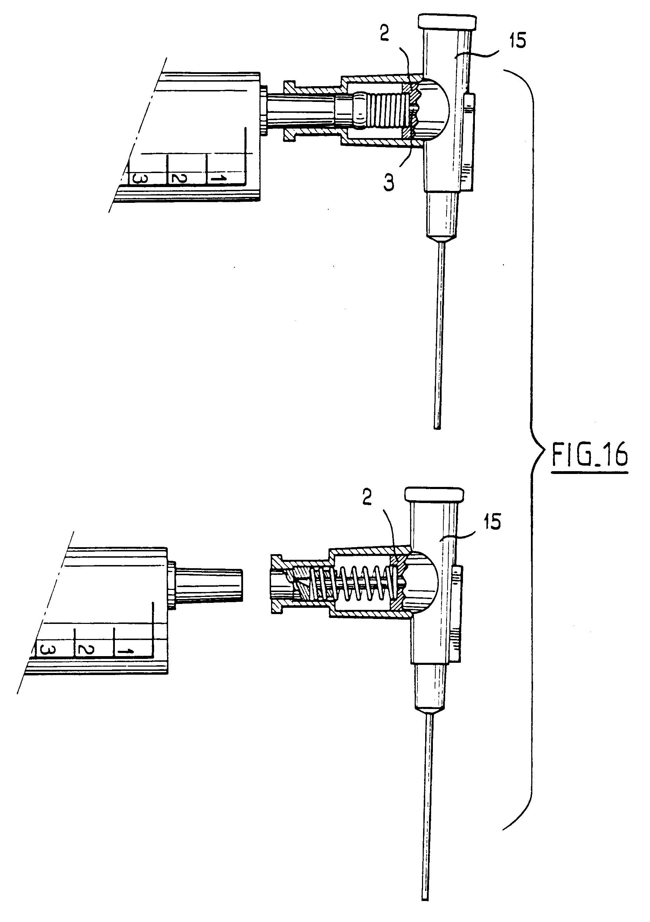

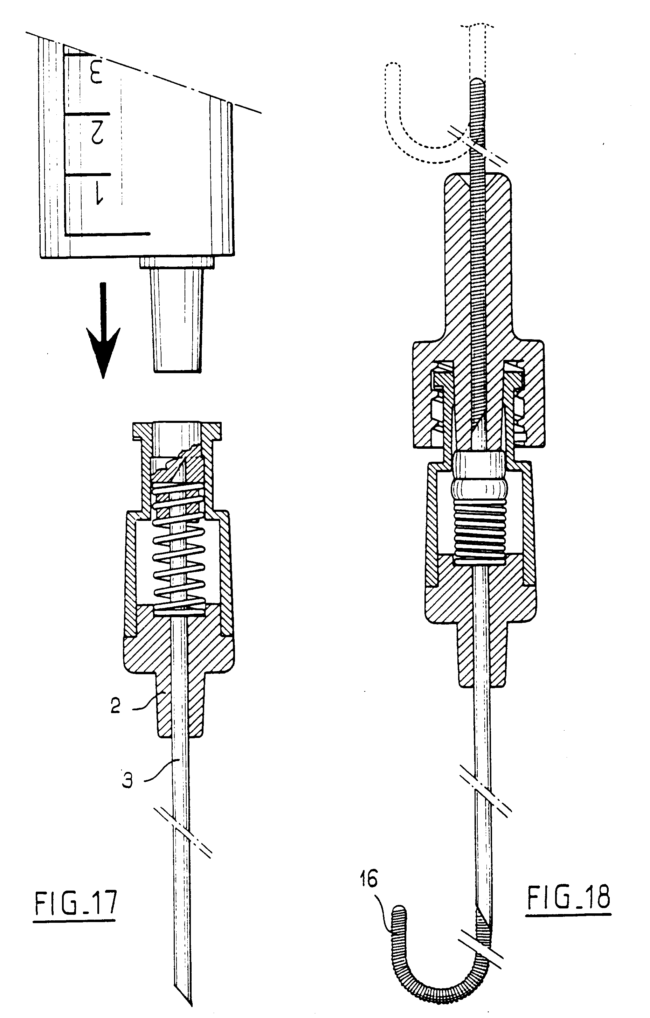

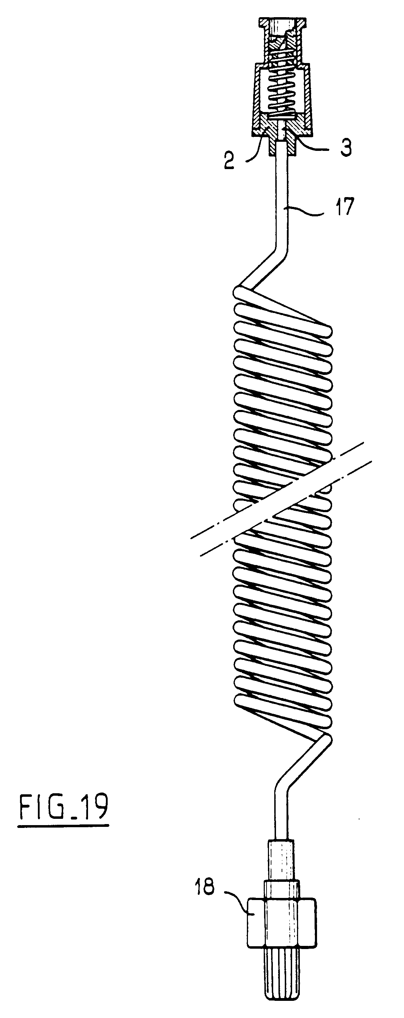

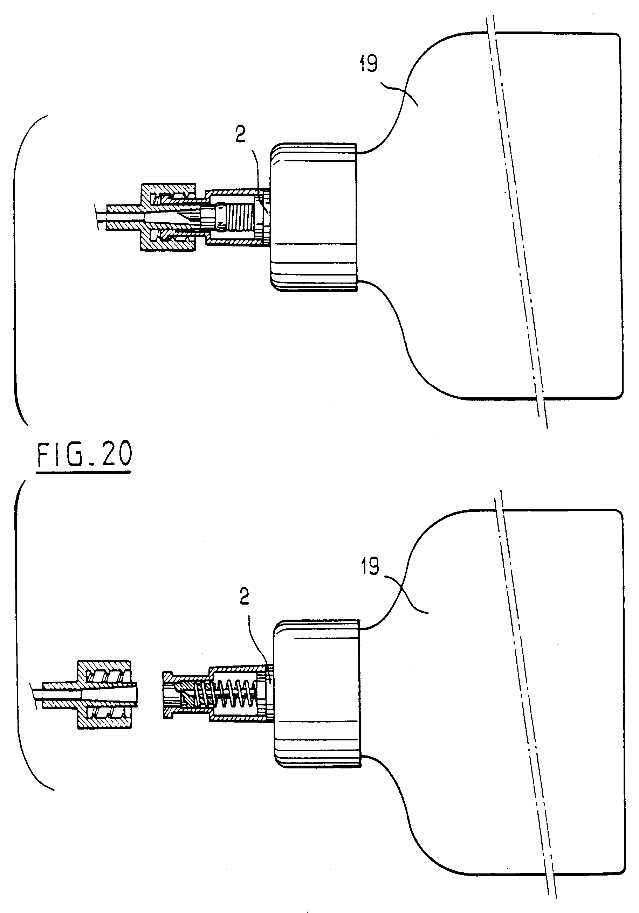

Dans ces réalisations, selon les cas :
- Le raccord amont 2 est du type Luer Lock Male (figure 13);
- Le connecteur est double et symétrique (figure 14): la chambre est divisée en deux compartiments respectivement aval et amont 6', 6'', l'aiguille 3 est tenue dans la chambre par un appui central 13 qu'elle traverse et présente deux extrémités opposées biseautées 10', 10'' dirigées respectivement vers le raccord aval 7 et vers le raccord amont 2, et deux bouchons mobiles 4', 4'', obturent la communication, respectivement entre le compartiment aval et le raccord aval et entre le compartiment amont et le raccord amont, chacun de ces bouchons étant soumis à l'action d'un ressort 5', 5'' qui prend appui sur l'appui central 13 et qui pousse le bouchon vers une position où il est en butée et n'est pas traversé par l'aiguille. Sur la figure 14, on a représenté à gauche le connecteur au repos, les deux bouchons étant en position d'obturation et, à droite le connecteur en service d'un côté, et au repos de l'autre côté ;
- Le raccord amont 2 est solidaire d'un tube 14 (figure 15);
- Le raccord amont 2 est solidaire d'un embout 15 qui constitue un conduit disposé transversalement par rapport à l'aiguille 3 (fig. 16);
- L'aiguille 3 traverse de part en part le raccord amont 2 (figure 17);
- L'aiguille traverse de part en part le raccord amont et reçoit un guide métallique interne 16 (figure 18) ;
- Le raccord amont 2 est solidaire d'un conduit souple tubulaire 17 dont une extrémité communique avec l'aiguille et dont l'extrémité opposée est munie d'un raccord 18 (figure 19).
- Le raccord amont 2 constitue un bouchon, par exemple le bouchon d'un flacon 19 (figure 20).

Les expressions "amont" et "aval" ont été utilisées pour désigner des positions relatives dans la direction qui va de l'aiguille au bouchon. En service, le liquide s'écoulera généralement en sens inverse en sorte que si l'on prenait comme direction de référence le sens d'écoulement du liquide, il faudrait inverser les expressions : le raccord aval devrait être désigné comme étant le raccord amont, l'ouverture de sortie du passage de ce raccord devrait être considérée comme une ouverture d'entrée, etc.

L'invention n'est pas limitée aux modes de réalisation qui ont été décrits.

## Revendications

1. Connecteur composite unitaire pour un circuit de liquide, notamment pour usage médical, qui comprend:
- des moyens (1,2) qui constituent une chambre tubulaire (6; 6',6'') entre un raccord amont (2) et un raccord aval (7) situés à des extrémités opposées de la chambre et fixes l'un par rapport à l'autre, ledit raccord aval constituant un passage (7b);
- une aiguille creuse (3 ; 3'), fixe dans la chambre, et apte à faire communiquer le raccord amont avec le raccord aval;
- un bouchon (4; 4') apte à être traversé par l'aiguille, monté dans le passage du raccord aval de façon à pouvoir coulisser entre une position aval stable d'obturation où il obture ledit passage et n'est pas traversé par l'aiguille et une position amont où il est traversé par ladite aiguille et vers laquelle il peut être poussé par un organe (S) introduit depuis l'extérieur du connecteur dans ledit passage, et un moyen (5, 5') situé dans la chambre et qui repousse élastiquement le bouchon vers la position stable d'obturation, la matière du bouchon étant telle que le bouchon recouvre ses propriétés d'obturation lorsqu'il n'est plus traversé par l'aiguille, caractérisé en ce que ledit passage (7b) du raccord aval (7) a une ouverture de sortie (7a) totalement remplie par la matière du bouchon (4) lorsque ce dernier est en position stable d'obturation, ce qui évite tout volume mort à l'extrémité du raccord et l'introduction de bactéries à cet endroit, et en ce que le moyen qui repousse élastiquement le bouchon est un ressort (5 ; 5').

2. Connecteur selon la revendication 1, caractérisé en ce que ledit bouchon (4) est introduit à force dans ledit passage (7b).

3. Connecteur selon la revendication 1 ou 2, caractérisé en ce que le bouchon (4) coulisse avec étanchéïté latérale dans ledit passage (7b) et dans ladite chambre (6).

4. Connecteur selon l'une des revendications 1 et 3, caractérisé en ce que la position stable d'obturation est définie par une butée (11) contre laquelle porte le bouchon.

5. Connecteur selon l'une des revendications 1 à 4, caractérisé en ce que ledit ressort (5) est logé dans la chambre (6) entre un appui qui porte l'aiguille et le bouchon (4), ce ressort poussant le bouchon vers sa position aval où le bouchon se trouve en butée contre un épaulement constitué par un rebord annulaire interne (11) de la chambre en amont dudit passage (7b).

6. Connecteur selon l'une des revendications 1 à 5, caractérisé en ce que le bouchon (4) présente une extrémité (4d) concave.

7. Connecteur selon l'une des revendications 1 à 5, caractérisé en ce que le bouchon (4) présente une extrémité (4d) convexe.

8. Connecteur selon l'une des revendications 1 à 7, caractérisé en ce que ledit passage (7b) du raccord aval présente une conicité.

9. Connecteur selon la revendication 8 caractérisé en ce que ledit bouchon (4) présente une conicité correspondant à celle dudit passage (7b).

10. Connecteur selon l'une des revendications 1 à 9, caractérisé en ce que ladite aiguille (3) comporte une extrémité biseautée.

11. Connecteur selon l'une des revendications 1 à 10, caractérisé en ce que la partie du bouchon qui doit être traversée par l'aiguille comporte une préperforation longitudinale (4e).

12. Connecteur selon l'une des revendications 1 à 11, caractérisé en ce que lorsque le bouchon (4) est en position d'obturation, l'extrémité de l'aiguille (3) est noyée dans la matière du bouchon.

13. Connecteur selon l'une des revendications 1 à 12, caractérisé en ce qu'il est constitué par la combinaison de cinq pièces, à savoir un carter (1), un raccord amont (2), une aiguille (3), un bouchon d'obturation (4) et un ressort hélicoïdal (5) assemblés en un ensemble unitaire.

14. Connecteur selon la revendication 13, caractérisé en ce que le carter (1) délimite une chambre tubulaire (6) entre une extrémité aval (7) qui est conformée pour constituer ledit passage et une extrémité amont (8 ) qui est conformée pour recevoir le raccord amont (2).

15. Connecteur selon l'une des revendications 1 à 14, caractérisé en ce que le raccord amont (2) est un raccord mâle.

16. Connecteur selon l'une des revendications 1 à 15, caractérisé en ce que la chambre est divisée en deux compartiments respectivement aval et amont (6', 6''), l'aiguille (3) est tenue dans la chambre par un appui (13) et présente deux extrémités opposées biseautées (10', 10'') dirigés respectivement vers le raccord aval (7) et vers le raccord amont (2), et en ce que deux bouchons mobiles (4', 4''), obturent la communication, respectivement entre le compartiment aval et le raccord aval et entre le compartiment amont et le raccord amont, chacun de ces bouchons étant soumis à l'action d'un ressort (5', 5'') qui prend appui sur l'appui central (13) et qui pousse le bouchon vers une position où il est en butée et n'est pas traversé par l'aiguille.

17. Connecteur selon l'une des revendications 1 à 12, caractérisé en ce que le raccord amont (2) est solidaire d'un tube (14).

18. Connecteur selon l'une des revendications 1 à 12, caractérisé en ce que le raccord amont (2 ) est solidaire d'un embout (15) qui constitue un conduit disposé transversalement par rapport à l'aiguille (3).

19. Connecteur selon l'une des revendications 1 à 12, caractérisé en ce que l'aiguille (3) traverse de part en part le raccord amont (2).

20. Connecteur selon l'une des revendications 1 à 12, caractérisé en ce que l'aiguille traverse de part en part le raccord amont et reçoit un guide métallique interne (16).

21. Connecteur selon l'une des revendications 1 à 12, caractérisé en ce que le raccord amont (2) est solidaire d'un conduit souple tubulaire (7) dont une extrémité communique avec l'aiguille et dont l'extrémité opposée est munie d'un raccord (18).

## Claims

1. A unitary composite connector for a liquid circuit, in particular for medical applications, the connector comprising:
means (1, 2) constituting a tubular chamber (6; 6', 6'') between an upstream coupling (2) and a downstream coupling (7) situated at opposite ends of the chamber and fixed relative to each other, said upstream coupling constituting a passage (7b);
a hollow needle (3; 3') which is fixed in the chamber and which is suitable for causing the upstream coupling to communicate with the downstream coupling; and
a plug (4; 4') suitable for being passed through by the needle, the plug being mounted in the passage of the downstream coupling so as to be capable of sliding between a downstream stable closure position where the plug closes said passage and where the needle does not pass through the plug, and an upstream position where said needle does pass through the plug and towards which the plug can be pushed by a member (S) inserted in said passage from outside said connector, and means (5, 5') situated in the chamber resiliently urging the plug towards its stable closure position, the material of the plug being such that the plug retrieves its closure properties when the needle is not passing through it, the connector being characterized in that said passage (7b) of the downstream coupling (7) has an outlet opening (7a) that is completely filled by the material of the plug (4) when the plug is in its stable closure position, thereby preventing any dead volume at the end of the coupling and preventing bacteria entering at this location, and in that the means resiliently urging the plug is a spring (5; 5').

2. A connector according to claim 1, characterized in that said plug (4) is a force-fit in said passage (7b).

3. A connector according to claim 1 or 2, characterized in that the plug (4) slides with lateral sealing in said passage (7b) and in said chamber (6).

4. A connector according to claim 1 or 3, characterized in that the stable, closure position is defined by an abutment (11) against which the plug bears.

5. A connector according to any one of claims 1 to 4, characterized in that said spring (5) is received in the chamber (6) between an abutment which carries the needle and the plug (4), said spring urging the plug towards its downstream position where the plug is in abutment against a shoulder constituted by an inside annular rim (11) of the chamber upstream from said passage (7b).

6. A connector according to any one of claims 1 to 5, characterized in that the plug (4) has a concave end (4d).

7. A connector according to any one of claims 1 to 5, characterized in that the plug (4) has a convex end (4d).

8. A connector according to any one of claims 1 to 7, characterized in that said passage (7b) of the downstream coupling is conical in shape.

9. A connector according to claim 8, characterized in that said plug (4) has a conical shape complementary to that of said passage (7b).

10. A connector according to any one of claims 1 to 9, characterized in that said needle (3) includes a chamfered end.

11. A connector according to any one of claims 1 to 10, characterized in that the portion of the plug through which the needle is to pass includes a longitudinal perforation (4e).

12. A connector according to any one of claims 1 to 11, characterized in that when the plug (4) is in its closure position, the end of the needle (3) is embedded in the material of the plug.

13. A connector according to any one of claims 1 to 12, characterized in that it is constituted by a combination of five pieces, namely: a case (1), an upstream coupling (2), a needle (3), a closure plug (4), and a helical spring (5), which pieces are assembled together to constitute a unitary assembly.

14. A connector according to claim 13, characterized in that the case (1) delimits a tubular chamber (6) between a downstream end (7) which is shaped to constitute said passage and an upstream end (8) which is shaped to receive the upstream coupling (2).

15. A connector according to any one of claims 1 to 14, characterized in that the upstream coupling (2) is a male coupling.

16. A connector according to any one of claims 1 to 15, characterized in that the chamber is divided into two compartments, respectively a downstream compartment (6') and an upstream compartment (6''), the needle (3) is held in the chamber by a support (13) and has two opposite chamfered ends (10', 10'') respectively pointing towards the downstream coupling (7) and towards the upstream coupling (2), and in that two moving plugs (4', 4'') close off communication respectively between the downstream compartment and the downstream coupling, and between the upstream compartment and the upstream coupling, each of said plugs being subjected to the action of a spring (5', 5'') which bears against the central support (13) and which pushes the plug towards a position where it is in abutment and the needle does not pass therethrough.

17. A connector according to any one of claims 1 to 12, characterized in that the upstream coupling (2) is secured to a tube (14).

18. A connector according to any one of claims 1 to 12, characterized in that the upstream coupling (2) is secured to an endpiece (15) which constitutes a duct disposed transversely relative to the needle (3).

19. A connector according to any one of claims 1 to 12, characterized in that the needle (3) passes right through the upstream coupling (2).

20. A connector according to any one of claims 1 to 12, characterized in that the needle passes right through the upstream coupling and receives an internal metal guide (16).

21. A connector according to any one of claims 1 to 12, characterized in that the upstream coupling (2) is secured to a tubular flexible duct (17) having one end communicating with the needle, and having its opposite end provided with a coupling (18).

## Patentansprüche

1. Einstückiges Verbindungselement für einen Flüssigkeitskreislauf, insbesondere für die medizinische Verwendung, das aufweist:
- Einrichtungen (1, 2), die eine rohrförmige Kammer (6; 6', 6'') zwischen einem stromaufwärtigen Anschluß (2) und einem stromabwärtigen Anschluß (7) bilden, welche an gegenüberliegenden Enden der Kammer angeordnet sind und mit in Bezug aufeinander befestigt sind, wobei der stromabwärtige Anschluß einen Durchlaß (7b) bildet;
- eine Hohlnadel (3; 3'), die in der Kammer befestigt ist, und so ausgelegt ist, daß sie die Verbindung zwischen dem stromaufwärtigen Anschluß und dem stromabwärtigen Anschluß herstellen kann;
- einen Stopfen (4; 4'), der von der Nadel durchquert werden kann, der derart in dem Durchlaß des stromabwärtigen Anschlusses angeordnet ist, daß er zwischen einer stromabwärtigen stabilen Verschlußposition, in der er den Durchlaß verschließt und nicht von der Nadel durchquert wird, und einer stromaufwärtigen Position, in der er von der Nadel durchquert wird und in die er durch ein Element (S) geschoben werden kann, das von außerhalb des Verbindungselementes her in den Durchlaß eingeführt wird, gleiten kann, und eine Einrichtung (5, 5'), die in der Kammer angeordnet ist und die in elastischer Weise den Stopfen in die stabile Verschlußposition zurückschiebt, wobei das Material des Stopfens derart ist, daß er seine Verschlußeigenschaften wiedergewinnt, wenn er nicht mehr von der Nadel durchquert wird, dadurch gekennzeichnet, daß der Durchlaß (7b) des stromabwärtigen Anschlusses (7) eine Ausgangsöffnung (7a) hat, die vollständig von dem Material des Stopfens (4) ausgefüllt ist, wenn dieser letztere sich in der stabilen Verschlußposition befindet, was jegliches tote Volumen am Ende des Anschlusses und das Einführen von Bakterien an dieser Stelle vermeidet, und daß die Einrichtung, die in elastischer Weise den Stopfen zurückschiebt, einen Feder (5; 5') ist.

2. Verbindungselement nach Anspruch 1, dadurch gekennzeichnet, daß der Stopfen (4) mit Kraft in den Durchlaß (7b) eingeführt ist.

3. Verbindungselement nach Anspruch 1 oder 2, dadurch gekennzeichnet, daß der Stopfen (4) seitlich dicht in dem Durchlaß (7b) und in der Kammer (6) gleitet.

4. Verbindungselement nach einem der Ansprüche 1 und 3, dadurch gekennzeichnet, daß die stabile Verschlußposition durch eine Anlage (11) definiert ist, an der der Stopfen anliegt.

5. Verbindungselement nach einem der Ansprüche 1 bis 4, dadurch gekennzeichnet, daß die Feder (5) in der Kammer (6) zwischen einer Anlage, die die Nadel trägt, und dem Stopfen (4) untergebracht ist, wobei diese Feder den Stopfen in seine stromabwärtige Position schiebt, in der der Stopfen sich in Anlage gegen eine Schulter befindet, die durch eine ringförmige Innenleiste (11) der Kammer stromaufwärts des Durchlasses (7) gebildet ist.

6. Verbindungselement nach einem der Ansprüche 1 bis 5, dadurch gekennzeichnet, daß der Stopfen (4) ein konkaves Ende (4d) zeigt.

7. Verbindungselement nach einem der Ansprüche 1 bis 5, dadurch gekennzeichnet, daß der Stopfen (4) ein konvexes Ende (4d) zeigt.

8. Verbindungselement nach einem der Ansprüche 1 bis 7, dadurch gekennzeichnet, daß der Durchlaß (7) des stromaufwärtigen Anschlusses eine Konizität zeigt.

9. Verbindungselement nach Anspruch 8, dadurch gekennzeichnet, daß der Stopfen (4) eine Konizität zeigt, die der des Durchlasses (7b) entspricht.

10. Verbindungselement nach einem der Ansprüche 1 bis 9, dadurch gekennzeichnet, daß die Nadel (3) ein geschliffenes Ende aufweist.

11. Verbindungselement nach einem der Ansprüche 1 bis 10, dadurch gekennzeichnet, daß der Teil des Stopfens, der von der Nadel durchquert werden soll, eine in Längsrichtung verlaufende Vorlochung (4e) hat.

12. Verbindungselement nach einem der Ansprüche 1 bis 11, dadurch gekennzeichnet, daß, wenn der Stopfen (4) in der Verschlußposition ist, das Ende der Nadel (3) in das Material des Stopfens eingetaucht ist.

13. Verbindungselement nach einem der Ansprüche 1 bis 12, dadurch gekennzeichnet, daß es aus der Kombination von fünf Teilen gebildet ist, nämlich einem Gehäuse (1), einem stromaufwärtigen Anschluß (2), einer Nadel (3), einem Verschlußstopfen (4) und einer Schraubenfeder (5), die zu einer einstückigen Gesamtheit zusammengesetzt sind.

14. Verbindungselement nach Anspruch 13, dadurch gekennzeichnet, daß das Gehäuse (1) eine rohrförmige Kammer (6) zwischen einem stromaufwärtigen Ende (7), das so gebildet ist, daß es den Durchlaß bildet, und einem stromabwärtigen Ende (8), das so gebildet ist, daß es den stromaufwärtigen Anschluß (2) aufnimmt, begrenzt.

15. Verbindungselement nach einem der Ansprüche 1 bis 14, dadurch gekennzeichnet, daß der stromaufwärtige Anschluß (2) männlicher Anschluß ist.

16. Verbindungselement nach einem der Ansprüche 1 bis 15, dadurch gekennzeichnet, daß die Kammer in zwei jeweils stromabwärtige und stromaufwärtige Abteile (6', 6'') aufgeteilt ist, wobei die Nadel (3) in der Kammer durch eine Anlage (13) gehalten ist und zwei geschliffene, einander gegenüberstehende Enden (10', 10'') zeigt, die jeweils gegen den stromaufwärtigen Anschluß (7) und gegen den stromabwärtigen Anschluß (2) gerichtet sind, und daß zwei bewegliche Stopfen (4', 4'') die Verbindung verschließen, jeweils zwischen dem stromabwärtigen Abteil und dem stromabwärtigen Anschluß und zwischen dem stromaufwärtigen Abteil und dem stromaufwärtigen Anschluß, wobei jeder der Stopfen der Wirkung einer Feder (5', 5'') ausgesetzt ist, die an einer mittleren Anlage (13) anliegt und die den Stopfen in eine Position schiebt, in der er anliegt und nicht von der Nadel durchquert wird.

17. Verbindungselement nach einem der Ansprüche 1 bis 12, dadurch gekennzeichnet, daß der stromaufwärtige Anschluß (2) einstückig mit einem Rohr (14) ausgebildet ist.

18. Verbindungselement nach einem der Ansprüche 1 bis 13, dadurch gekennzeichnet, daß der stromaufwärtige Anschluß (2) mit einem Ansatzstück (15) ausgebildet ist, das eine Leitung bildet, welche quer in bezug auf die Nadel (3) angeordnet ist.

19. Verbindungselement nach einem der Ansprüche 1 bis 12, dadurch gekennzeichnet, daß die Nadel (3) den stromaufwärtigen Anschluß (2) vollständig durchquert.

20. Verbindungselement nach einem der Ansprüche 1 bis 12, dadurch gekennzeichnet, daß die Nadel vollständig den stromaufwärtigen Anschluß durchquert und eine innere metallische Führung (16) aufnimmt.

21. Verbindungselement nach einem der Ansprüche 1 bis 12, dadurch gekennzeichnet, daß der stromaufwärtige Anschluß (2) einstückig mit einer rohrförmigen, weichen Leitung (17) ausgebildet ist, deren eines Ende mit der Nadel kommuniziert und dessen gegenüberliegendes Ende mit einem Anschluß (18) versehen ist.
